# EUROPEAN PATENT SPECIFICATION

(11) **EP 1 415 663 B1**
(45) Date of publication and mention of the grant of the patent: **15.10.2008**
(21) Application number: 02746046.8
(22) Date of filing: 15.07.2002
(51) Int. Cl.: A61K 38/22, A61K 9/06, A61K 9/08, A61K 47/42, A61K 47/48, A61P 9/00, A61P 43/00

(54) **SUSTAINED RELEASE HGF HYDROGEL PREPARATIONS**
HGF-HYDROGEL-ZUBEREITUNGEN MIT VERZÖGERTER FREISETZUNG
PREPARATIONS D'HYDROGEL HGF A LIBERATION PROLONGEE

(30) Priority: 18.07.2001 JP 2001218870
(43) Date of publication of application: 06.05.2004
(73) Proprietor: MedGEL Corporation, Kyoto-shi Kyoto 612-8043 (JP)
(72) Inventor: Tabata, Yasuhiko, Kyoto 611-0024 (JP)
(74) Representative: von Kreisler, Alek
(86) International application number: PCT/JP2002/007155
(87) International publication number: WO 2003/007982

(56) References cited:
- WO-A-01/28577
- WO-A2-01/22989
- JP-A- 8 295 634
- US-A- 5 059 213
- US-A- 6 036 972
- YAMAMOTO M. ET AL.: 'Controlled release of growth factors based on biodegradation of gelatin hydrogel' J. BIOMATER. SCI. POLYM. ED. vol. 12, no. 1, January 2001, pages 77 - 88, XP002957328

## Description

### Technical Field

The present invention relates to a sustained release HGF gelatin hydrogel preparation obtained by combining the hepatocyte growth factor (HGF) and a gelatin hydrogel having specific properties. More particularly, the present invention relates to a complex of HGF and a gelatin hydrogel, an HGF impregnated gelatin hydrogel preparation, an angiogenesis and vasculogenesis promoter utilizing them, a therapeutic agent for arterial diseases.

### Background Art

The hepatocyte growth factor (HGF) is a protein found as a potent growth promoting factor of mature hepatocytes, which has been genetically cloned (Biochem Biophys Res Commun, 122, 1450 (1984), Proc. Natl. Acad. Sci, USA, 83, 6489, (1986), FEBS Letter, 22, 311 (1987), Nature, 342, 440 (1989), Proc. Natl. Acad. Sci, USA, 87, 3200 (1990)). Subsequent studies have clarified that HGF not only works as a liver regeneration factor *in vivo* for the repair and regeneration of diseased liver, but also has an angiogenetic and vasculogenetic action and plays a major role in the prophylaxis or treatment of ischemic diseases and arterial diseases (Symp. Soc. Exp. Biol., 47 cell behavior, 227-234 (1993), Proc. Natl. Acad. Sci., 90, 1937-1941 (1993), Circulation, 97, 381-390 (1998)).

As mentioned above, HGF has various functions including the function of an angiogenesis and vasculogenesis factor and many attempts have been made toward enhanced use of HGF as a pharmaceutical product. However, what has become a problem here was the half-life of HGF in blood. The half-life of HGF is as short as several minutes to 10 minutes, which makes it difficult to maintain blood concentration and to ensure transfer of an effective amount of HGF to the affected area. Therefore, mere injection of the protein in the form of a solution is considered to fail to afford a sufficient physiological activity of HGF, because the protein is rapidly diffused and excreted from the injection site.

The only method considered to enhance effectiveness *in vivo* is to impregnate a polymer carrier with HGF to achieve sustained release of HGF for a long time. In recent years, some tests have demonstrated that certain growth factors such as basic fibroblast growth factor, bone-inducing protein, transformation growth factor and the like, show anticipated *in vivo* physiological activity when used in combination with various carrier matrices [Downs, E.C. et al (1992), Miyamoto, S. et al (1992), Gombotz, W.R. et al (1993)]. However, there is no report relating to HGF release *in vivo,* except a few study results that reveal that an anticipated physiological action is induced when a physiologically excessive dose of an HGF solution is injected [Matsuda, Y. et al (199)].

The present inventor has already conducted various studies directed to the sustained release of these growth factors, based on the finding that bFGF, TGF-β1 and HGF interact with acidic polysaccharides, such as heparan sulfate, heparin and the like, in the extracellular matrix (ECM) and are preserved in the body [Taipale, J., Keski-Oja, J. (1997), Mizuno, K. et al (1994), Arakaki, N. et al (1992), Sakata, H. et al (1997)]. In the case of "basic" bFGF and TGF-β1, the present inventor has heretofore prepared a biodegradable hydrogel from an "acidic" gelatin having an isoelectric point of 5.0 and capable of ionic interaction, and prepared hydrogels impregnated with bFGF and TGF-β1. These impregnated hydrogels can release a physiologically active growth factor in a sustained manner due to the decomposition in the living organism. The released growth factor shows an *in vivo* physiological action and the duration of the activity matched well with the growth factor release time [Tabata, Y., Ikada, Y. (1999)].

However, which gelatin hydrogel shows a preferable combination with HGF was not clear, and, as the situation stood, production of a gelatin hydrogel preparation of HGF was desired.

The present invention aims at providing a sustained release HGF gelatin hydrogel preparation. More particularly, the present invention relates to a complex of HGF and a gelatin or gelatin hydrogel, an angiogenesis and vasculogenesis promoter utilizing the same, a therapeutic agent for arterial diseases.

The present inventor has conducted intensive studies of a sustained release HGF gelatin hydrogel preparation. To be specific, the effect of sustained release of HGF and gelatin hydrogel was first studied to clarify that a remarkable effect of sustained release can be provided only in the combination of a gelatin hydrogel having specific properties and HGF.

It has been also clarified that a complex of HGF and gelatin hydrogel is formed at a specific molar ratio (molar ratio of HGF:gelatin constituting hydrogel of 7:1). Furthermore, it has been found that a complex of HGF and gelatin is similarly formed and HGF in the complex is stable to proteases such as trypsin and the like.

Because this HGF gelatin (hydrogel) complex is less affected by the ionic strength in the solution, it has been clarified that not only static interaction but also other actions such as hydrophobic binding greatly contribute to the complex. For example, in the case of bFGF, which is a protein other than HGF, a bFGF gelatin hydrogel complex is similarly formed, but this complex is greatly affected by the ionic strength and shows further suppressed bFGF sorption to gelatin with increasing ionic strengths in the solution. In the case of HGF, however, the effect of ionic strength is smaller as compared to bFGF.

In addition, the present inventor prepared chemically crosslinked gelatins containing various amounts of glutaraldehyde using a gelatin suitable for the present invention, prepared various hydrogels having different degradabilities, and studied the effects of the hydrogels. For example, it has been clarified that, when a ¹²⁵I-labeled HGF impregnated gelatin hydrogel is prepared and implanted in the body of a mouse, HGF radioactivity remains in the subcutaneous tissues of the mouse for a long time with increasingly elevating glutaraldehyde concentrations. As demonstrated, the time profile of HGF remaining in each gelatin hydrogel matches well with the time profile of hydrogel degradation, which clearly indicates release of HGF by biodegradation of hydrogel.

Furthermore, an HGF impregnated gelatin hydrogel was prepared using a gelatin gel preferable for the present invention and implanted in a mouse. As a result, histological changes by angiogenesis and vasculogenesis were induced around the implanted hydrogel. To be specific, by implanting gelatin hydrogel preparations containing 5 and 10 µg of HGF, the number of capillaries newly formed around the implanted area has been found to increase significantly.

The present invention has been completed based on the above-mentioned findings.

### Disclosure of the Invention

Accordingly, the present invention provides the following.
1. A sustained release HGF gelatin hydrogel preparation comprising a gelatin hydrogel obtained by crosslinking a gelatin having the following properties and a hepatocyte growth factor (HGF):
   (1) an acidic gelatin obtained from collagen by an alkali hydrolysis treatment,
   (2) a molecular weight of 100,000 - 200,000 daltons under non-reducing conditions of SDS-PAGE,
   (3) an isoelectric point of about 5, and
   (4) a zeta potential in an aqueous solution of -15 to -20 mV, wherein the HGF is contained at a notice of not less than 0,7 mol and not more than 7 mol per one mol of the gelatin.
2. The sustained release HGF gelatin hydrogel preparation of the above-mentioned 1, wherein the gelatin hydrogel has a water content of 80 - 99 w/w%.
3. The sustained release HGF gelatin hydrogel preparation of the above-mentioned 1 or 2, which comprises the HGF at a ratio of not more than 7 mol per one mol of the gelatin constituting the gelatin hydrogel.
4. An HGF stabilized gelatin preparation comprising an HGF and a gelatin having the following properties:
   (1) an acidic gelatin obtained from collagen by an alkali hydrolysis treatment,
   (2) a molecular weight of 100,000 - 200,000 daltons under non-reducing conditions of SDS-PAGE,
   (3) an isoelectric point of about 5, and
   (4) a zeta potential in an aqueous solution of -15 to -20 mV, wherein the HGF is contained at a notice of not less than 0,7 mol and not more than 7 mol per one mol of the gelatin.
5. An HGF gelatin complex comprising an HGF wherein the HGF is contained at a notice of not less than 0,7 mol and not more than 7 mol per one mol of the gelatin. having the following properties:
   (1) an acidic gelatin obtained from collagen by an alkali hydrolysis treatment,
   (2) a molecular weight of 100,000 - 200,000 daltons under non-reducing conditions of SDS-PAGE,
   (3) an isoelectric point of about 5, and
   (4) a zeta potential in an aqueous solution of -15 to -20 mV.
6. An aqueous HGF stabilized gelatin preparation comprising the HGF gelatin complex of the above-mentioned 5.
7. An angiogenesis and vasculogenesis promoter comprising a preparation selected from the group consisting of the sustained release HGF gelatin hydrogel preparations of the above-mentioned 1 to 3, the HGF stabilized gelatin preparation of the above-mentioned 4, the HGF gelatin complex of the above-mentioned 5 and the aqueous HGF stabilized gelatin preparation of the above-mentioned 6.
8. A therapeutic agent for treating arterial diseases, which comprises a preparation selected from the group consisting of the sustained release HGF gelatin hydrogel preparations of the above-mentioned 1 to 3, the HGF stabilized gelatin preparation of the above-mentioned 4, the HGF gelatin complex of the above-mentioned 5 and the aqueous HGF stabilized gelatin preparation of the above-mentioned 6.

### Brief Description of the Drawings

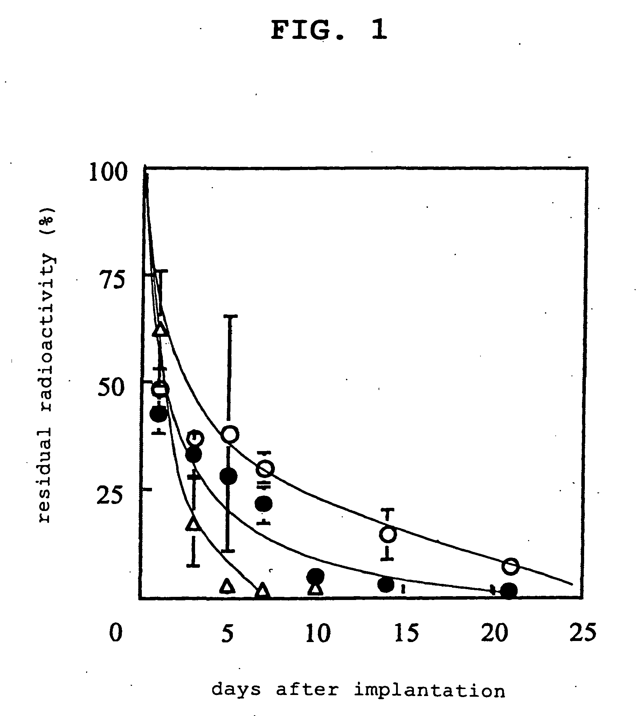

Fig. 1 shows progress in time of residual radioactivity after subcutaneous implantation of a ¹²⁵I-labeled hydrogel [gel 1 (○), gel 2 (●) or gel 3 (Δ)] in the back of a mouse, wherein the line is obtained according to the method of least squares.

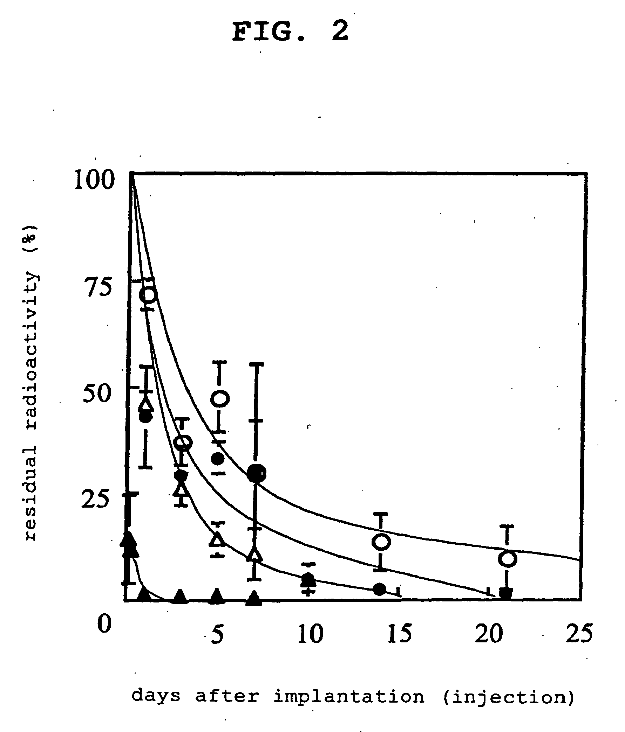

Fig. 2 shows progress in time of residual radioactivity after subcutaneous implantation of gel 1 (○), gel 2 (●) and gel 3 (Δ), which are impregnated with ¹²⁵I-labeled HGF, in the back of mice or after subcutaneous injection (▲) of ¹²⁵I-labeled HGF in the back of a mouse in the form of a solution, wherein the line is obtained according to the method of least squares.

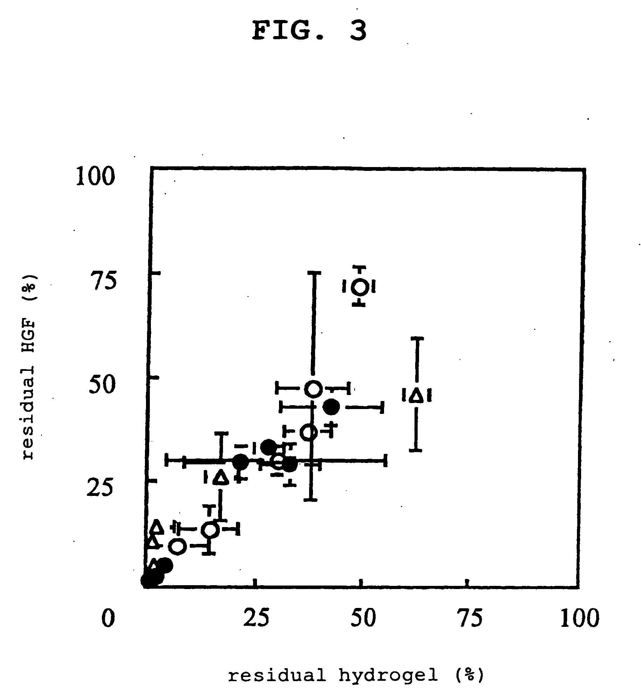

Fig. 3 shows the relationship in the residual radioactivity between ¹²⁵I-labeled HGF impregnated gelatin hydrogel and ¹²⁵I-labeled gelatin hydrogel after subcutaneous implantation in the back of mice, wherein the hydrogel was prepared from gel 1 (○), gel 2 (●) or gel 3 (Δ).

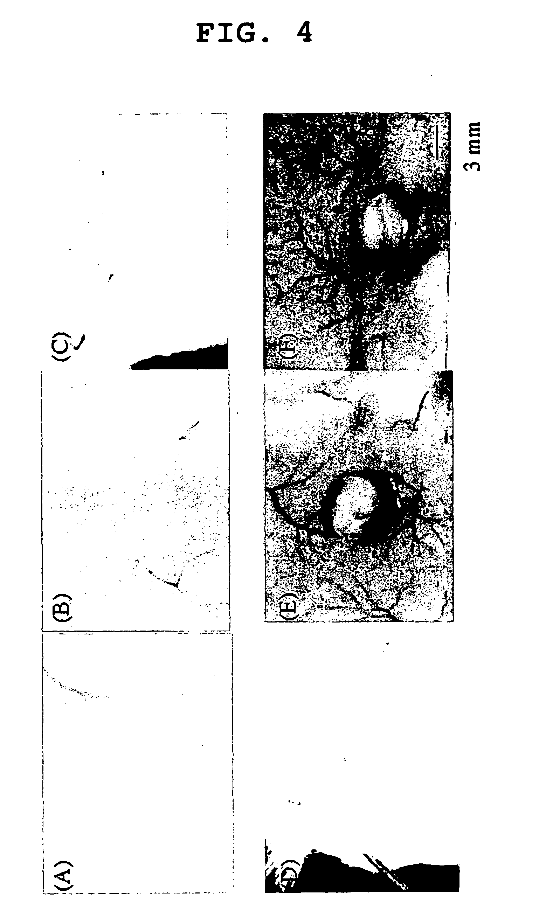

Fig. 4 shows subcutaneous tissue characteristics of each mouse at day 7 after injection of PBS (A) free of HGF and free HGF 10 µg (B), or after implantation of gel 2 hydrogel (C) without impregnating HGF and free of anything else and HGF 1 µg (D), 5 µg (E) and 10 µg (F) impregnated gel 2 hydrogels.

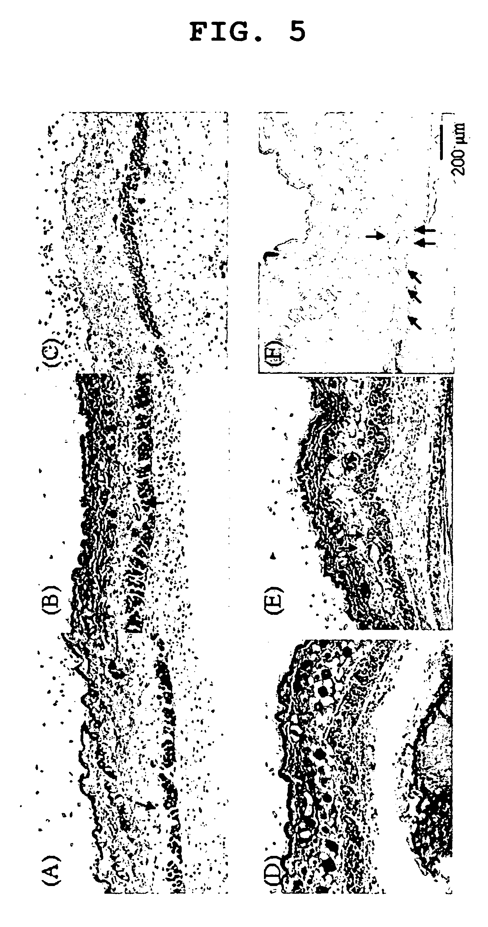

Fig. 5 shows histological section of each mouse at day 7 after injection of PBS (A) free of HGF and free HGF 10 µg (B), or after implantation of gel 2 hydrogel (C) without impregnating HGF and free of anything else and HGF 1 µg (D), 5 µg (E) and 10 µg (F) impregnated gel 2 hydrogels, wherein arrows show newly formed blood vessels.

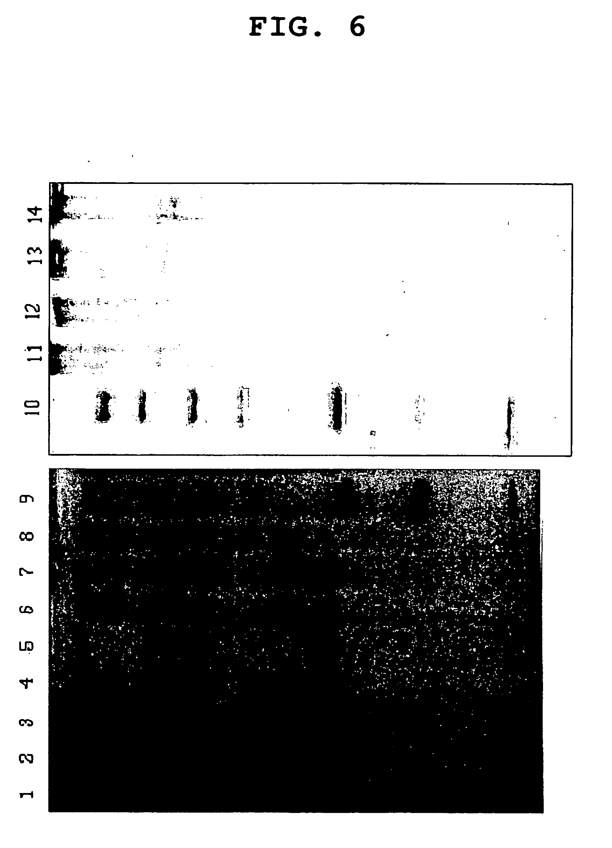

Fig. 6 shows the results of gel electrophoresis exhibiting the stabilization effect of HGF against trypsin by formation of a complex with gelatin, wherein lanes 1, 9, 10 are standard proteins, lane 2 is gelatin (Nitta Gelatin Inc.; isoelectric point 9.0), lane 3 is HGF+gelatin (Nitta Gelatin Inc.; isoelectric point 9.0), lane 4 is HGF+gelatin (Nitta Gelatin Inc.; isoelectric point 9.0) after incubation at 37°C for 24 hr, lane 5 is HGF alone, lane 6 is HGF+gelatin (Nitta Gelatin Inc.; isoelectric point 5.0) after incubation at 37°C for 24 hr, lane 7 is HGF+gelatin (Nitta Gelatin Inc.; isoelectric point 5.0), lane 8 is gelatin (Nitta Gelatin Inc.; isoelectric point 5.0), lane 11 is gelatin (Sigma, type A), lane 12 is gelatin (manufactured by Wako Pure Chemical Industries, Ltd.), lane 13 is gelatin (Nitta Gelatin Inc.; isoelectric point 9.0), and lane 14 is gelatin (Nitta Gelatin Inc.; isoelectric point 5.0).

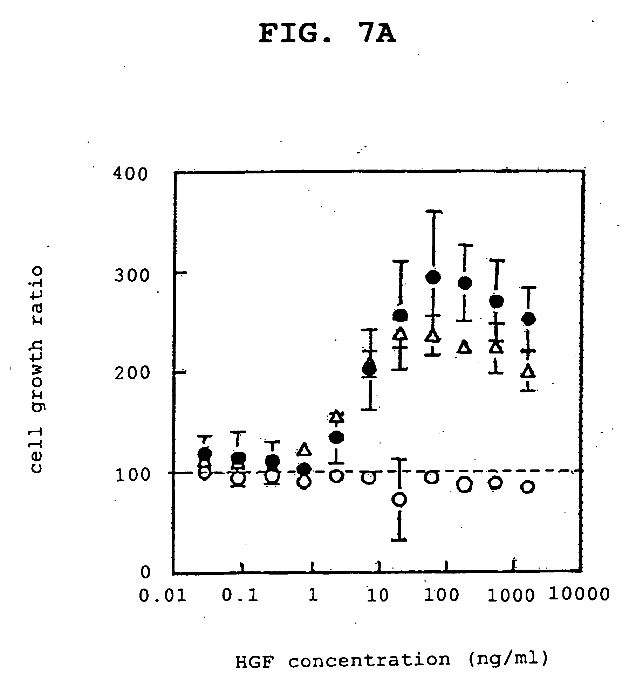

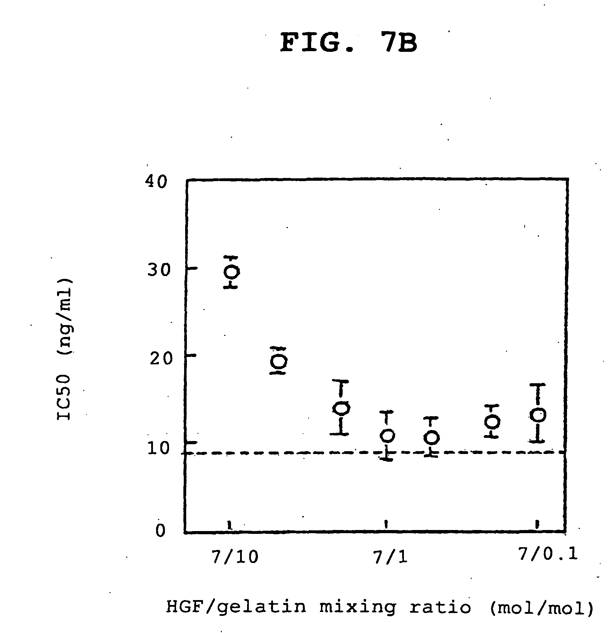

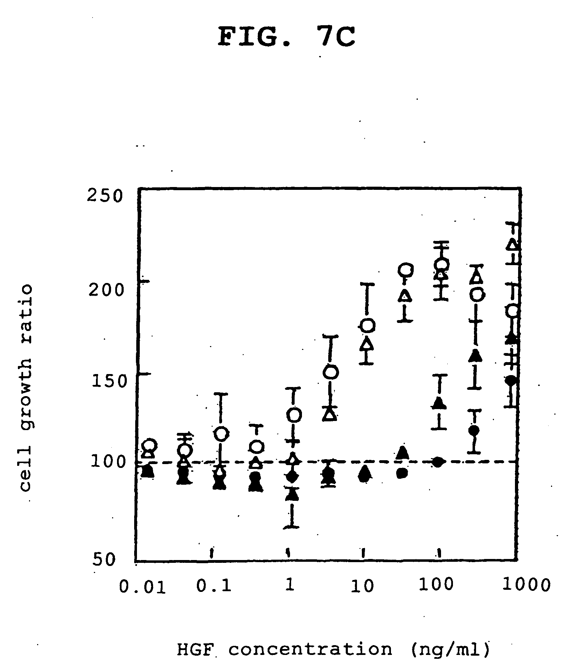

Fig. 7 (A, B and C) shows the effect of mixing with gelatin on the cell growth promoting activity of HGF. Fig. 7A shows the relationship between the HGF concentration and cell growth activity of free HGF (●), gelatin (○) and HGF (Δ) mixed with acidic gelatin. Fig. 7B shows the effect of HGF/gelatin mixing ratio on the cell growth promoting activity of HGF mixed with gelatin. Fig. 7C shows the relationship between cell growth rate and HGF concentration in HGF treated with trypsin (●) or not treated with trypsin (○), and HGF+gelatin treated with trypsin (▲) or not treated with trypsin (Δ).

### Detailed Description of the Invention

The gelatin to be used in the present invention has the following properties and is different from commercially available gelatin:
(1) an acidic gelatin obtained from collagen by an alkali hydrolysis treatment,
(2) a molecular weight of 100,000 - 200,000 daltons under non-reducing conditions of SDS-PAGE,
(3) an isoelectric point of about 5, and
(4) a zeta potential in an aqueous solution of -15 to -20 mV.

The commercially available gelatin includes, for example, type A gelatin manufactured by Sigma and gelatin manufactured by Wako Pure Chemical Industries, Ltd., that have different zeta potentials in an aqueous solution as shown in the following:

| | |
|---|---|
| type A gelatin manufactured by Sigma: | about 0 to about 5 mV |
| gelatin manufactured by Wako Pure Chemical Industries, Ltd.: | about -5 to about -2 mV |

The zeta potential is a scale showing the static charge of a substance (gelatin), which is considered to be preferable as an index of gelatin that forms an electrostatic complex with HGF in the present invention.

The gelatin in the present invention is obtained by alkali hydrolysis of a part of the skin, tendon and the like or collagen of various animal species including cow. Preferably, it is an acidic gelatin prepared by an alkali treatment of type I collagen derived from a bone of cow, or may be obtained as a sample IEP5.0 from Nitta Gelatin Inc. A basic gelatin prepared by an acid treatment is also available from Nitta Gelatin Inc. as sample IEP9.0, but the zeta potential differs vastly as shown below:

| | |
|---|---|
| acidic gelatin (Nitta Gelatin Inc. sample IEP5.0): | about -15 to about -20 mV |
| basic gelatin (Nitta Gelatin Inc. sample IEP9.0): | about +12 to about +15 mV |

The gelatin hydrogel to be used in the present invention is a hydrogel obtained by condensation with various chemical crosslinking agents using the above-mentioned gelatin. As the chemical crosslinking agent, for example, glutaraldehyde, water-soluble carbodiimides such as 1-ethyl-3-(3-dimethylaminopropyl)carbodiimide hydrochloride, 1-cyclohexyl-3-(2-morpholinoethyl)carbodiimide metho-p-toluenesulfonate, bisepoxy compound, formalin are preferable, and glutaraldehyde and 1-ethyl-3-(3-dimethylaminopropyl)carbodiimide hydrochloride are particularly preferable.

The gelatin can be also crosslinked by a heat treatment or ultraviolet irradiation.

The shape of gelatin hydrogel is free of any particular limitation and may be, for example, columnar, rectangle columnar, sheet, disc, spherical, granularlar. Columnar, rectangle columnar, sheet and disc hydrogels are often used generally as a graft for transplantation, and spherical or granular hydrogels may be also used for injection.

Columnar, rectangle columnar, sheet and disc gelatin hydrogels can be prepared by adding an aqueous solution of a crosslinking agent to an aqueous gelatin solution, or by adding a gelatin to an aqueous solution of a crosslinking agent, pouring the solution into a mold having a desired shape and allowing a crosslinking reaction to take place. An aqueous solution of a crosslinking agent may be added to the formed gelatin gel as it is or after drying. The crosslinking reaction can be stopped by contacting the solution with a low molecular weight substance having an amino group, such as ethanolamine, glycine, or adding an aqueous solution having a pH of 2.5 or below. The obtained gelatin hydrogel is washed with distilled water, ethanol, 2-propanol, acetone and used for producing a preparation.

Spherical and granular gelatin hydrogels can be prepared by, for example, placing a gelatin solution in a device wherein a three-mouthed round-bottom flask is equipped with a fixed stirring motor (e.g., manufactured by Shinto Scientific Co., Ltd., Three One Motor, EYELA mini D.C. Stirrer etc.) and a Teflon (trademark) stirring propeller and fixed together, adding an oil such as olive oil, stirring the mixture at a rate of about 200 - 600 rpm to give a W/O type emulsion and adding an aqueous solution of a crosslinking agent thereto, or adding dropwise a previously pre-emulsified aqueous gelatin solution in olive oil (e.g., using a vortex mixer Advatec TME-21, homogenizer Polytron PT10-35 etc) to olive oil, preparing a minute particle W/O type emulsion and adding an aqueous solution of a crosslinking agent thereto, thereby to allow for a crosslinking reaction, recovering a gelatin hydrogel by centrifugation, washing the gelatin hydrogel with acetone, ethyl acetate and immersing the gelatin hydrogel in 2-propanol, ethanol to stop the crosslinking reaction. The obtained gelatin hydrogel particles are washed successively with 2-propanol, distilled water containing Tween 80, distilled water and used to give a preparation.

When gelatin hydrogel particles coagulate, for example, ultrasonication (preferably under cooling, within about one minute) may be performed.

By pre-emulsification, a gelatin hydrogel, a minute particle gelatin hydrogel having a particle size of 20 µm or below can be obtained.

The average particle size of the obtained gelatin hydrogel particles (value obtained by a conventional method from a microscopic photograph of particles swollen with water by measuring the particle size of at least 400 particles and calculating an average) is 1 - 1000 µm, and particles having a desired size may be appropriately screened and used according to the object.

As another method for preparing a spherical or granular gelatin hydrogel, the following method can be also mentioned.

Olive oil is placed in a device similar to the one used for the above-mentioned method, stirred at a rate of about 200 - 600 rpm, an aqueous gelatin solution is added dropwise thereto to give a W/O type emulsion, which is cooled, stirred with acetone, ethyl acetate, and centrifuged to recover gelatin particles. The recovered gelatin particles are washed with acetone, ethyl acetate, then with 2-propanol, ethanol and dried. This dry gelatin particles are suspended in an aqueous solution of a crosslinking agent, which contains 0.1% Tween 80, the suspension is gently stirred to allow for crosslinking reaction, washed with 100 mM aqueous glycine solution containing 0.1% Tween 80 or 0.004N HCl containing 0.1% Tween 80, depending on the crosslinking agent used, to stop the crosslinking reaction, whereby a gelatin hydrogel particle can be prepared. The average particle size of the gelatin hydrogel particle obtained by this method is the same as that in the above-mentioned method.

The conditions of the crosslinking reaction are free of any particular limitation and are, for example, 0 - 40°C, 1 - 48 hr.

It has been clarified with regard to the gelatin hydrogel of the present invention that its water content greatly affects the sustained releasability of HGF. The water content that shows a preferable effect of sustained release is 80 - 99 w/w%. More preferable water content is 95 - 98 w/w%.

The gelatin hydrogel of the present invention is appropriately cut into a suitable size, lyophilized, sterilized and used. The lyophilization includes, for example, placing a gelatin hydrogel in distilled water, freezing the hydrogel in liquid nitrogen for not shorter than 30 min or at -80°C for not shorter than 1 hr, and drying for 1 - 3 days in a lyophilizer.

When gelatin hydrogel is prepared, the concentrations of gelatin and crosslinking agent may be appropriately determined depending on desired water contents. For example, the concentration of gelatin is 1 - 20 w/w% and the concentration of the crosslinking agent is 0.001 - 1 w/w%.

When gelatin hydrogel was prepared using commercially available gelatin such as type A gelatin manufactured by Sigma or gelatin manufactured by Wako Pure Chemical Industries, Ltd., the effect of sustained release of HGF could not be achieved even by the use of hydrogel impregnated with HGF. When the above-mentioned acidic gelatin of the present invention was used to prepare the above-mentioned gelatin hydrogel, the effect of sustained release of HGF could be achieved.

The HGF to be used in the present invention is a known substance, and as long as it is purified to the degree that permits use as a pharmaceutical agent, those prepared by various methods can be used, and the products already on the market (e.g., Toyobo Code No. HGF-101) may be used. For production of HGF, for example, primary culture cell or established cell line capable of producing HGF is cultured, and HGF can be obtained from culture supernatant by separation and purification. Alternatively, a gene encoding HGF is incorporated into a suitable vector by genetic engineering, the vector is inserted into a suitable host for transformation, and the object recombinant HGF can be obtained from culture supernatant of the transformant (e.g., Nature, 342, 440 (1989), JP-A-5-111383, Biochem. Biophys. Res. Commun. 163, 967 (1989)). The above-mentioned host cell is not particularly limited, and various host cells, such as *Escherichia coli*, yeast, animal cell, which have been conventionally used for genetic engineering, can be used. The HGF thus obtained may have an amino acid sequence containing one or more amino acids that are substituted, deleted and/or added, and a sugar chain may be similarly substituted, deleted and/or added, as long as it has substantially the same action with naturally occurring HGF.

The sustained release HGF gelatin hydrogel preparation of the present invention is obtained by impregnating the above-mentioned acidic gelatin hydrogel with HGF. In addition, the HGF stabilized gelatin preparation of the present invention comprises HGF and the above-mentioned acidic gelatin. Since HGF is a basic protein, it forms a complex with an acidic gelatin (hydrogel) in a solution. In view of the aforementioned effect of HGF to suppress sorption due to changes in the ionic strength in a solution, however, this HGF gelatin (hydrogel) complex is based not only on an electrostatic interaction but also greatly on other interactions such as hydrophobic binding. The dissociation constant (Kd) and the binding molar ratio of HGF to gelatin of this complex were obtained according to the Scatchard binding model [Scatchard, G. (1949)]. As the binding molar ratio of HGF to gelatin, it has been shown that 7 HGF molecules bind with one acidic gelatin molecule. When compared to bFGF that forms a similar complex, bFGF gelatin complex has a 1:1 molar ratio, which is strikingly different from that of HGF.

The Kd value of acidic gelatin at 37°C is 5.5×10⁻⁷ M, and this value is about 2 - 3 orders greater than the Kd value of heparin sulfate of 1×10⁻⁹ - 2.0×10⁻¹⁰ M at 20°C [Rahmoune H et al (1998)]. This suggests that the binding performance of HGF gelatin complex is not as strong as that of HGF heparin sulfate and moderate.

When the molar ratio of HGF to gelatin is raised to 1:7 or above, HGF is easily liberated and the activity behavior is almost the same as that of free HGF. However, when the molar ratio of HGF is lowered to 1:7 or below, HGF is adsorbed and a less amount of HGF is liberated, which reduces the apparent activity of HGF.

Therefore, while it is possible to make many having various molar ratios of HGF and gelatin, as a complex of HGF and gelatin or gelatin hydrogel, a preferable complex to avoid an initial burst has a molar ratio of 7 mol or below of HGF per one mol of gelatin (or gelatin consisting of gelatin hydrogel).

As regards gelatin, one having a weight ratio of HGF of not more than 5-fold amount is preferable. More preferable one has a weight ratio of HGF to gelatin of 5 - 1/10⁴-fold amount.

Since the sustained release HGF gelatin hydrogel preparation of the present invention has an effect of sustained release of HGF and an HGF stabilization effect, it can exhibit the function of HGF for a long time with a small amount. Consequently, since it effectively exhibits an angiogenetic and vasculogenetic action, which is the inherent function of HGF, the sustained release HGF gelatin hydrogel preparation of the present invention can be effectively used as an angiogenesis and vasculogenesis promoter, or a therapeutic agent of arterial diseases (e.g., arteriosclerosis, ischemic reperfusion injury, cardiac infarction, angina pectoris, intractable arterial diseases, Buerger's disease, posttransplantation arteriosclerosis) in mammals (e.g., cat, cow, dog, horse, goat, monkey, human).

The HGF stabilized gelatin preparation of the present invention has an HGF protecting effect of gelatin based on an HGF gelatin electrostatic complex, as in the case of the gelatin hydrogel preparation, and can be present stably against proteinases such as trypsin. Based on this stabilization effect, and from the aspects of easy use as an aqueous solution, it can be used parenterally as a preparation for injection. For example, it can be administered subcutaneously, intramuscularly, intravenously, intracoelomically or to diseased organ. Accordingly, the HGF stabilized gelatin preparation of the present invention can be effectively used as an angiogenesis and vasculogenesis promoter, a therapeutic agent of arterial diseases or a therapeutic agent of organ disorders in mammals (e.g., cat, cow, dog, horse, goat, monkey, human).

An HGF stabilized gelatin preparation in the form of an aqueous solution (aqueous HGF stabilized gelatin preparation) contains the above-mentioned HGF gelatin complex (preferably one having a weight ratio of HGF of 5-fold amount or less relative to gelatin) in water for injection or various buffers (e.g., phosphate buffer, carbonate buffer, HEPES buffer, Tris buffer). The concentration of the HGF gelatin complex in the preparation is not particularly limited and can be appropriately adjusted according to the disease to be treated, age and body weight of patients, dose. When, for example, this preparation is administered to an adult patient at a dose of 1 - 10 ml, the concentration of the HGF gelatin complex in the preparation is generally in the range of 0.001 - 500 µg/ml, preferably 0.1 - 200 µg/ml, as the concentration of HGF.

The sustained release HGF gelatin hydrogel preparation or complex of the present invention can take an appropriate dosage form suitable for each use. For example, it can be administered in the dosage form of a sheet, a stick, a particle, a rod or a paste.

As the administration method, intradermal administration, subcutaneous administration, intramuscular administration, intracavity administration can be mentioned.

The preparation of the present invention can contain pharmaceutically acceptable conventional additives (stabilizer, preservative, solubilizer, pH adjusting agent, thickener) where necessary. They may be known substances. In addition, various additives (low molecular weight substances such as amino acid, sugar having amino group or phosphoric acid group, sulfuric acid group, SH group, carboxyl group, lipid, high molecular weight substances) that control the sustained release effect and other active ingredients having an action to enhance the effect of HGF, an action to suppress decomposition and/or inactivation of HGF. Such other active ingredients include any low molecular weight substances and high molecular weight substances such as polysaccharide, lipid, glycolipid, protein, sugar protein, peptide, various low molecular weight compounds or high molecular weight compounds usable as a drug.

While the dose of the preparation of the present invention can be appropriately controlled according to the disease to be treated, age and body weight of patients and the like, it is generally in the range of 0.01 - 500 µg, preferably 1 - 200 µg, as the amount of HGF, for an adult patient, which is injected into the diseased area or in the vicinity thereof. When a single administration is not sufficient in effect, the dose may be given plural times.

The present invention is explained in detail in the following by referring to Examples.

### Example 1

### Preparation of HGF impregnated gelatin hydrogel preparation

### (1) Preparation of gelatin hydrogel

Using a gelatin (manufactured by Nitta Gelatin Inc. sample IEP 5.0) obtained by alkali treatment and preparation of type I collagen derived from bovine bone, a 5 wt% aqueous gelatin solution (50 ml) was prepared, and mixed with aqueous glutaraldehyde solutions (25 wt%; 40, 50 and 70 µl) to achieve the final concentrations of 5.00, 6.25 and 8.75 mM. The mixed solution was poured into a plastic mold (8×8 cm², 6 mm thick) and left standing at 4°C for 18 hr to allow for crosslinking of gelatin. Thereafter, the obtained gelatin hydrogel sheet was immersed in a 100 mM aqueous glycine solution at 37°C for 1 hr to block aldehyde residue of glutaraldehyde. The crosslinked hydrogel sheet was cut into an about 10 mg disc (diameter 6 mm), which was washed 3 times with double distilled water (DDW) at 37°C and lyophilized. The preparation conditions and water contents of gelatin hydrogel are summarized in Table 1. The weight of the hydrogel before and after swelling in DDW at 37°C for 24 hr was measured and water content of hydrogel, which shows a weight ratio of water therein relative to wet hydrogel, was calculated. Higher glutaraldehyde concentrations showed lower hydrogel water contents. The lyophilized hydrogel was sterilized with ethylene oxide gas. The hydrogel did not show changes in shape before and after the lyophilization and sterilization processes.

**Table 1 Characteristics of prepared gelatin hydrogel**

| code | gelatin concentration (wt%) | glutaraldehyde concentration (mM) | water content (wt%) |
|---|---|---|---|
| Gel 1 | 5 | 8.75 | 93.8 |
| Gel 2 | 5 | 6.25 | 94.8 |
| Gel 3 | 5 | 5.00 | 96.0 |

### (2) Preparation of HGF impregnated gelatin hydrogel

Using phosphoric acid bufferized physiological saline (PBS, pH 7.4) containing 1, 5 or 10 µg of HGF (manufactured by PeproTech EC. Ltd., London UK), lyophilized gelatin hydrogel disc was impregnated with HGF. A hydrogel free of impregnation with HGF and containing nothing else was produced by the same method except that PBS free of HGF was used. Briefly describing, PBS (20 µl) containing or not containing HGF was added dropwise onto a lyophilized gelatin hydrogel and left standing overnight at 4°C. As a result, an HGF impregnated gelatin hydrogel was obtained irrespective of the kind of hydrogel. Because the amount of this solution was considerably smaller than the theoretical amount to be impregnated into each hydrogel, the lyophilized gelatin hydrogel completely sorbed the aqueous HGF solution in a swelling step. In the same manner as above, an aqueous solution of ¹²⁵I-labeled HGF was sorbed in a lyophilized gelatin hydrogel disc to prepare a ¹²⁵I-labeled HGF impregnated gelatin hydrogel. The ¹²⁵I-labeled HGF was prepared according to the Chloramine T method heretofore reported [Lyon, M. et al (1994)]. Each of the prepared hydrogel discs showed the same appearance (shape and size) irrespective of the kind and radioactive label of hydrogel.

### Example 2

### Preparation of HGF stabilized gelatin preparation

### (1) Preparation of HGF gelatin complex (7:1 molar ratio)

Using the gelatin of Example 1, a 74 µg/ml solution was prepared. Production included preparing a 14.7 µg/ml solution of HGF (manufactured by Genzyme/Techne), separating 5 µl thereof, adding it to 5 µl of an aqueous gelatin solution, stirring the mixture and standing the mixture at 37°C for 24 hr.

### (2) Preparation of HGF gelatin electrostatic complex other than 7:1 molar ratio

For production, the aqueous gelatin solution and HGF aqueous solution of the above (1) were mixed at a different ratio and prepared to achieve a desired molar ratio and stood at 37°C for 24 hr.

### Reference Example 1

### Property measurement of gelatin

### (1) Measurement of isoelectric point

A 1 w/w% aqueous solution of acidic gelatin (Nitta Gelatin Inc. sample IEP5.0) was prepared and this aqueous solution was filtered through a cation column and an anion column. The pH of the filtered aqueous solution was measured. As the measurement value, pH of about 4.9 - 5.0 was obtained. This value was taken as an isoelectric point of gelatin.

### (2) Measurement of zeta potential

A gelatin was dissolved in water for millique treatment to give a 0.1 mg/ml aqueous solution. This aqueous solution was measured using ELS (ELS-7000AS, manufactured by OTSUKA ELECTRONICS CO., LTD.). The measure temperature was 25 - 27°C and ELS standard cell was used.

The results are shown in the following.

| | |
|---|---|
| [gelatin sample] | [zeta potential] |
| acidic gelatin (Nitta Gelatin Inc. sample IEP5.0): | about -15 to about -20 mV |
| basic gelatin (Nitta Gelatin Inc. sample IEP9.0): | about +12 to about +15 mV |
| type A gelatin manufactured by Sigma: | about 0 to about 5 mV |
| gelatin manufactured by Wako Pure Chemical Industries, Ltd.: | about -5 to about -2 mV |

### Reference Example 2

### in vivo degradation profile of gelatin hydrogel

### (1) Radioactive labeling of gelatin hydrogel

The gelatin hydrogel of Example 1 was labeled with radioactive iodine using a ¹²⁵I-Bolton-Hunter reagent [Bolton, A.E., Hunter, W.M. (1963)]. For evaporation of benzene, dry nitrogen gas was blown to a solution (100 µl) of ¹²⁵I-Bolton-Hunter reagent in dry benzene until benzene completely evaporated. Then, 0.1M sodium borate buffer (125 µl, pH 8.5) was added to the dry reagent to give an aqueous solution of ¹²⁵I-Bolton-Hunter reagent. The prepared aqueous solution (20 µl per disc) was impregnated into a gelatin hydrogel lyophilized disc. The resulting swollen hydrogel was maintained at 4°C for 3 hr, whereby the ¹²⁵I residue was introduced into an amino group of the gelatin. By exchanging DDW at fixed intervals at 4°C for 4 days, unbound liberated ¹²⁵I-labeling reagent was removed from the ¹²⁵I-labeled gelatin hydrogel. As a result of the measurement at fixed intervals, the radioactivity of DDW returned to the background level after washing for 3 days. Irrespective of the hydrogel preparation conditions, the swollen hydrogel disc did not show changes in the shape during radioactive labeling and the subsequent washing step. The swollen hydrogel obtained finally was lyophilized.

### (2) Evaluation of in vivo degradation of gelatin hydrogel

The *in vivo* degradation of gelatin hydrogel was evaluated from the attenuation of the radioactivity of the ¹²⁵I-labeled gelatin hydrogel implanted in the body. Various ¹²⁵I-labeled gelatin hydrogels were implanted into subcutaneous tissues of the back of ddY mice (3 mice/group, 6 - 7 weeks of age, Shizuoka Animal Center, Shizuoka Prefecture). At days 1, 3, 5, 7, 10, 14 and 21 of hydrogel implantation, the radioactivity of the implanted hydrogels was measured using a gamma counter (ARC-301B, ALOKA CO., LTD., Tokyo). Then, the skin of the mice around the area of hydrogel implantation was removed in a small piece of 3×5 cm², the fascia area was completely wiped with a filter to absorb the eluted ¹²⁵I-labeled gelatin. The radioactivity of the skin piece and filter was measured, and the residual radioactivity of the tissues around the implanted hydrogel was evaluated. The ratio of the total radiation dose relative to the radioactivity of the hydrogel implanted first showed a ratio of the remaining activity due to hydrogel degradation. For each test group, 21 mice were used, and unless particularly described, 3 mice were killed for each time of evaluation performed for the *in vivo* evaluation. All animal tests were performed according to the in school guidance of Kyoto University relating to animal tests.

The results are shown in Fig. 1. Fig. 1 shows the progress with time of the residual gelatin radioactivity after subcutaneous implantation of ¹²⁵I-labeled gelatin hydrogel into the back of mice. Higher concentrations of glutaraldehyde used for hydrogel preparation show presence of residual radioactivity for a longer period. In every hydrogel, the residual radioactivity attenuated with the lapse of time. For example, a gelatin hydrogel having the highest water content was decomposed in 7 days of implantation into the body, but a hydrogel having the lowest water content was maintained for 21 days in the body.

As mentioned above, higher glutaraldehyde concentrations were associated with slower degradation of gelatin hydrogel. In general, higher glutaraldehyde concentrations used for hydrogel preparation mean higher crosslinking degrees of hydrogel. It is considered, therefore, that the degree of crosslinking of hydrogel stereochemically reduces the hydrolytic approach of protease to gelatin chain, and as a result, makes the sensitivity to protein hydrolysis lower.

### Example 3

### Evaluation of in vivo release of HGF from HGF impregnated gelatin hydrogel

Various gelatin hydrogels impregnated ¹²⁵I-labeled HGF were implanted into subcutaneous tissues of the back of mice. An aqueous solution of ¹²⁵I-labeled HGF (100 µl/area) was subcutaneously injected in the back of the mice. Then, the skin of the mice including the area of implanted hydrogel or injection area was removed at different time intervals, and the fascia area was completely wiped with a filter according to the method mentioned above. The residual radioactivity of the gelatin hydrogel, skin piece and filter was measured with a gamma counter. The ratio of the radiation relative to HGF before use was taken as a ratio of the remaining activity due to *in vivo* HGF release.

The results are shown in Fig. 2. Fig. 2 shows attenuation patterns of HGF radioactivity after subcutaneous implantation of ¹²⁵I-labeled HGF impregnated gelatin hydrogel into the back of mice. The residual radioactivity of HGF decreased with the lapse of time, irrespective of the kind of hydrogel. The attenuation pattern of the radioactivity was greatly dependent on the kind of hydrogel. Higher concentrations of glutaraldehyde for crosslinked hydrogel show presence of radioactivity for a longer period. In contrast, the radioactivity after injection of ¹²⁵I-labeled free HGF disappeared from the injection area within one day.

Fig. 3 shows the residual radioactivity of HGF as a relational factor of residual radioactivity of hydrogel. Irrespective of the kind of hydrogel, fine correlation was found between two residual radioactivities, wherein the slope was about 1.

From the above, by comparison of Fig. 1 with Fig. 2, the HGF radioactivity showed rapid attenuation in hydrogel, which is degraded rapidly. The presence of fine correlation in the attenuation patterns of HGF hydrogel radioactivity (Fig. 3) means release of HGF from the gelatin hydrogel in the body along with biodegradation of hydrogel. The adsorption test has shown interaction of HGF with acidic gelatin. It is considered that, once interacted with acidic gelatin, HGF molecule cannot be released from hydrogel unless a water-soluble gelatin fragment is formed by hydrogel degradation.

### Example 4

### Quantitative determination of HGF sorbed by gelatin hydrogel

To make an isotherm of HGF adsorption to hydrogel, an aqueous solution (0.5 ml) containing various amounts of ¹²⁵I-labeled HGF was added to hydrogel previously swollen with DDW (1 ml). HGF adsorption proceeded at 37°C over 48 hr, which taught that this was sufficient for equilibrium HGF adsorption, irrespective of HGF concentration. By measuring radiation, the equilibrium concentration (Cf) of free HGF in the solution was calculated. Taking the molar weights of HGF and gelatin to be 90,000 and 100,000, the molar ratio (r) of HGF adsorbed to gelatin was calculated. According to the Scatchard binding model [Scatchard, G. (1949)], r/Cf was plotted. The dissociation constant (Kd) and binding molar ratio of HGF relative to gelatin were obtained from the slope and intercept of r/Cf - r line, r = 0.

The results of HGF adsorption to gelatin are shown in the following.

Because the isotherm of HGF adsorption to gelatin hydrogel was the adsorption isotherms of Langmuir (data not shown), the adsorption parameter was calculated based on the Scatchard analysis. The Kd value of acidic gelatin at 37°C was 5.5×10⁻⁷M, which was greater by about 2 - 3 orders than the Kd value of heparin sulfate at 20°C of 1×10⁻⁹ - 2.0×10⁻¹⁰ M [Rahmoune H et al. (1998)]. This shows that the affinity of HGF for gelatin is.extremely low. About 7 molecules of HGF were bound with one molecule of acidic gelatin.

### Example 5

### in vitro bioassay of HGF

The physiological activity was evaluated with respect to the effect of HGF that promotes in vitro growth of Mv1Lu cells or HGF that formed a complex with gelatin [Tajima, H. et al (1992)]. As culture medium, Eagle medium (containing 10% fetal calf serum (FCS) and penicillin-streptomycin (GIBCO catalog No. 15140-122) Nissui) was used. The culture medium (100 µl) was placed in a 96 multiwell culture plate (Corning Corster) and incubated at 37°C for 1 hr under 5% CO₂-95% air atmosphere. Then, an aqueous HGF solution (50 ml) or an HGF-gelatin complex prepared at HGF concentration of 1.25 µg/ml was added to the well, the medium (50 µl, 150 µl) containing each HGF sample was sequentially transferred to the next well and 11 wells were prepared by 3-fold serial dilution. The mixture of gelatin and HGF was set to 37°C in DDW. Briefly describing, the HGF/gelatin mixing ratio was set to 14.2/1 and 7.1/1, aqueous solutions having various concentrations were left standing for 1 and 12 hr to give various HGF-gelatin complexes. Three wells were used for each HGF sample at every HGF concentration. Finally, a cell suspension (100 µl, 1×10⁴ cells/ml) was inoculated to each well. After incubation for 72 hr, cell growth was measured using a cell counter kit-8 (Dojindo Molecular Technologies, Inc., Bethesda, MD, USA). A kit-8 solution (10 µl) was added to each well, and the cells were incubated at 37°C for 4 hr. Using a UV max Kinetic Plate Reader (Molecular Devices Co., Menlo Park, CA, USA), the sorption of each well at wavelength of 450 nm was measured. The sorption of each sample well was compared with the sorption of control free of HGF relative to the latter (% cell growth). The HGF concentration capable of inducing 50% of the maximum cell growth of each HGF sample is expressed as GC₅₀.

The results are shown in Table 2. Table 2 summarizes GC₅₀ values of HGF and HGF-gelatin complex. While the range of GC₅₀ value scarcely depends on the HGF/gelatin mixing ratio and mixing time, GC₅₀ value increases by forming a gelatin complex. When a complex is formed with gelatin at 37°C, the dose-reaction curve of HGF shifts toward high dose, in parallel to the original dose-reaction curve of HGF. The gelatin per se does not have a cell growth-promoting action (data not shown).

**Table 2 Physiological activity of HGF and HGF mixed with acidic gelatin**

| | HGF/gelatin mixing ratio (mol/mol) | Mixing time (hr) | GC₅₀ (ng/ml) |
|---|---|---|---|
| HGF alone | - | - | 15.35 |
| HGF mixture | 7.1 | 1 | 117.9 |
| | | 12 | 178.0 |
| | 14.2 | 1 | 122.8 |
| | | 12 | 103.7 |

As shown above, Table 2 indicates that HGF shows physiological activity even when a complex is formed with acidic gelatin. The smaller activity of HGF can be explained from the formation of complex with gelatin. It is considered that the action of HGF on the cell surface receptor is smaller than that of free HGF because HGF forms a complex with gelatin.

### Example 6

### in vivo evaluation of angiogenesis and vasculogenesis introduced by HGF impregnated gelatin hydrogel

Any of gelatin hydrogels impregnated with 1, 5 and 10 µg of HGF and gelatin hydrogel not impregnated with HGF and not containing anything was implanted into the body in the subcutaneous tissue of the back of a mouse. As a control, PBS containing or not containing HGF (10 µg) was subcutaneously injected into the back of a mouse. Each test group contained 6 mice per one test point. The mouse was killed at day 7 of the implantation or injection to the body, and evaluation of HGF-induced angiogenesis and vasculogenesis seen in the subcutaneous tissue of the back was performed. The evaluation of the angiogenesis and vasculogenesis by HGF was based on the property, histological test and biochemical test of the tissue. First, subcutaneous tissues around HGF implantation area or injection area were photographed, and an angiogenetic and vasculogenetic action was evaluated by visual observation. Then three mice were randomly selected from 6 mice and skin specimens containing implanted hydrogel or injection area were fixed with 10 wt% neutralized formaldehyde solution, embedded in paraffin and sections (5 µm thick) were prepared. The prepared sections were subjected to hematoxylin-eosin staining and observed histologically under an optical microscope. Using three histological sections obtained from three mice for each test group, the number of capillaries newly formed in the implanted areas of HGF impregnated gelatin hydrogel or gelatin hydrogel free of HGF, as well as around the HGF and PBS injection areas were evaluated. Briefly describing, fixed areas around the implanted area and injection area (3 areas/section) were randomly observed and the capillaries were counted.

The remaining 3 mice were used for biochemical test of HGF induced angiogenesis and vasculogenesis. The skin piece (2×2 cm²) around the hydrogel implanted area or injection area was cut into small pieces and treated with 0.1M Tris-HCl and 0.2 mM EDTA solution (1 ml) at 4°C for 24 hr to extract a protein component. After centrifugation (12000 rpm, 10 min, 4°C), the concentration of vascular endothelial cell growth factor (VEGF) in the obtained supernatant was clarified by ELISA.

A report has documented that the cell activated by HGF secreted VEGF and the VEGF mediated the angiogenetic and vasculogenetic action of HGF [Wojta, J. et al (1999)].

The results are shown in Fig. 4 and Table 3. Fig. 4 shows the tissue property of the skin on the back after implantation of gelatin hydrogel impregnated with different doses of HGF or injection of PBS solution containing HGF. Changes caused by angiogenesis and vasculogenesis were observed around the implanted area of HGF (5 and 10 µg)-impregnated gelatin hydrogel, which seemed to be completely opposite from the free HGF injection area even at a high dose. HGF injection in the form of a solution did not affect the vascular distribution of the subcutaneous tissues, and the tissue observation of the injection area was similar to the observation of the HGF-free PBS injected mouse. With gelatin hydrogel alone, which was free of HGF, angiogenesis and vasculogenesis did not induce changes. Fig. 5 shows this tissue section. When HGF impregnated gelatin hydrogel was implanted, new blood vessels were formed around the implanted area and the area grew bigger as the dose of HGF increased. Both a gelatin hydrogel not impregnated with HGF and the highest dose of free HGF were ineffective. Harmful responses and severe inflammatory responses due to HGF-impregnated implanted hydrogel or HGF non-impregnated implanted hydrogel were not observed.

**Table 3 Number of capillary newly formed around implanted area of HGF impregnated gelatin hydrogel or free HGF injection area**

| | number of capillary (mm⁻²) |
|---|---|
| HGF impregnated gelatin hydrogel | |
| (HGF 10 µg/mouse) | 7.9 ± 2.9*^{,†} |
| (HGF 5 µg/mouse) | 8.3 ± 3.8*^{,†} |
| (HGF 1 µg/mouse) | 4.5 ± 1.9 |
| free HGF | |
| (HGF 10 µg/mouse) | 1.5 ± 0.6 |
| gelatin hydrogel not containing HGF and not containing anything else | 4.4 ± 1.8 |
| PBS | 2.6 ± 1.1 |

| | |
|---|---|
| *p<0.05; significance relative to free HGF administration group ^{†}p<0.05; significance relative to empty gelatin hydrogel implantation group | |

Table 3 shows the number of capillary newly formed around the HGF impregnated gelatin hydrogel implanted area. This number of capillary significantly increased due to gelatin hydrogel containing HGF (5 and 10 µg) as compared to other test groups. Induction of angiogenesis and vasculogenesis did not occur at free HGF 10 µg, and the number of capillary was the same as that with PBS free of HGF and gelatin hydrogel containing nothing else

**Table 4 Concentration of VEGF induced in subcutaneous tissue of mouse at day 7 from implantation of HGF impregnated gelatin hydrogel or injection of free HGF**

| | VEGF (µ mol/area) |
|---|---|
| HGF impregnated gelatin hydrogel | |
| (HGF 10 µg/mouse) | 1.59 ± 0.89 |
| (HGF 5 µg/mouse) | 2.18 ± 0.33 |
| (HGF 1 µg/mouse) | N.D. |
| free HGF | |
| (HGF 10 µg/mouse) | N.D. |
| gelatin hydrogel not containing HGF and not containing anything else | N.D. |
| PBS | N.D. |

| | |
|---|---|
| N.D.: not detected | |

Table 4 shows VEGF concentration in mouse subcutaneous tissue after implantation of HGF impregnated gelatin hydrogel or injection of free HGF. At day 7 of the injection, HGF (10 µg) in the PBS solution did not induce VEGF in the injection area, and the results were almost the same as those with PBS injection without HGF or those in untreated normal mouse. However, gelatin hydrogel impregnated with HGF (5 and 10 µg) caused increase in the VEGF concentration around hydrogel at day 7 of the implantation, which was opposite from the gelatin hydrogel containing nothing.

As mentioned above, it is clear from Table 3 that HGF impregnated gelatin hydrogel induces angiogenesis and vasculogenesis more significantly than the same amount of free HGF. A report has documented that the cell activated by HGF secreted VEGF and the VEGF mediated the angiogenetic and vasculogenetic action of HGF [Wojta, J. et al (1999)]. However, induction of VEGF was observed even on day 7 of implantation of the gelatin hydrogel impregnated with HGF (5 and 10 µg) (Table 4). The amount of 1 µg was not sufficient to maintain concentration of induced VEGF for a long time. From all accounts, the data indicate that, even if exposed to *in vivo* environment, HGF impregnated in the gelatin hydrogel maintains its physiological activity. It is considered that HGF released from hydrogel due to decomposition induces angiogenesis and vasculogenesis *in vivo.*

### Example 7

### Stability of HGF gelatin preparation against trypsin

To the HGF gelatin preparation of Example 2 was added 5 µl of an aqueous trypsin solution (2.3 µg/ml), and the mixture was thoroughly stirred and stood at 37°C for 3 hr. An aqueous trypsin inhibitor solution (5 µl, 3.5 µg/ml) was added, and the mixture was thoroughly stirred and stood at 37°C for 1 hr. This was subjected to SDS-PAGE using 10% acrylamide gel (20 mA, 90 min). As the control, an aqueous gelatin solution alone, an aqueous HGF solution alone, a solution free of trypsin and a trypsin inhibitor, and an HGF solution added with trypsin and a trypsin inhibitor were simultaneously electrophoresed. The results are shown in Fig. 6. The HGF gelatin preparation of the present invention was almost free of decomposition of HGF even by the trypsin treatment (lanes 4 and 6).

### Example 8

### Biological activity of HGF gelatin preparation

The HGF gelatin preparation of Example 2 was added to DMEM culture solution containing fetal calf serum. Mv1Lu cells (mink lung epithelial cell line) were cultured in the mixture. As a control, an aqueous HGF solution and an aqueous gelatin solution were used.

After culture for 3 days in culture solutions having different HGF concentrations, the grown cells were counted using a Cell Counting Kit (manufactured by DOJINDO LABORATORIES), and the cell growth rate was calculated in percentage relative to the number of cells grown without the addition of HGF. The HGF concentration, which is 1/2 of the maximum cell growth rate when cell growth rate is plotted against HGF concentration, was taken as IC50 (ng/ml). The biological activity of HGF was evaluated from this value or cell growth rate. HGF mixed with gelatin or HGF was digested with trypsin. Then, the cell growth rate of HGF was evaluated in the same manner to examine resistance of HGF to enzyme digestion, which was achieved by mixing with gelatin. All data were analyzed by the Dunnett's Multiple Comparison Method, and statistical significance was acknowledged at P < 0.05. The test results are shown for average ± standard deviation (SD) of average. The results are shown in Fig. 7.

Fig. 7A shows the cell growth activity of free HGF, gelatin and HGF mixed with acidic gelatin. Irrespective of the mixing with gelatin, the cell growth efficiency increased with increasing HGF concentrations, but the increase was reduced somewhat by mixing with gelatin. The gelatin itself did not show a cell growth promoting action. Fig. 7B shows the effect of the mixing ratio of HGF/gelatin on the cell growth promoting activity of HGF mixed with gelatin. When the mixing ratio was 7/1 or below, the cell growth promoting activity of HGF decreased. This is considered to be attributable to a decrease in the affinity of HGF for cell surface receptor due to the interaction with gelatin. Fig. 7C shows the effect of the trypsin treatment on the cell growth promoting activity of HGF. Mixing with gelatin showed an improved resistance of HGF to trypsin digestion. This is considered to have resulted from the covering of HGF with gelatin, which has made HGF molecule not easily attacked by trypsin.

### Industrial Applicability

According to the present invention, a gelatin sustained release preparation of HGF can be provided, and using this preparation, an angiogenesis and vasculogenesis promoter, a therapeutic agent for arterial diseases can be provided. The angiogenesis and vasculogenesis promoter and the therapeutic agent for arterial diseases of the present invention are applicable to many patients, and are expected to be useful as effective therapeutic preparations of HGF.

## Claims

1. A sustained release HGF gelatin hydrogel preparation comprising a gelatin hydrogel obtained by crosslinking a gelatin having the following properties and a hepatocyte growth factor (HGF):
(1) an acidic gelatin obtained from collagen by an alkali hydrolysis treatment,
(2) a molecular weight of 100,000 - 200,000 daltons under non-reducing conditions of SDS-PAGE,
(3) an isoelectric point of about 5, and
(4) a zeta potential in an aqueous solution of -15 to -20 mV, wherein the HGF is contained at a ratio of not less than 0.7 mol and not more than 7 mol per one mol of the gelatin constituting the gelatin hydrogel.

2. The sustained release HGF gelatin hydrogel preparation of claim 1, wherein the gelatin hydrogel has a water content of 80 - 99 w/w%.

3. An HGF stabilized gelatin preparation comprising an HGF an a gelatin having the following properties:
(1) an acidic gelatin obtained from collagen by an alkali hydrolysis treatment,
(2) a molecular weight of 100,000 - 200,000 daltons under non-reducing conditions of SDS-PAGE,
(3) an isoelectric point of about 5, and
(4) a zeta potential in an aqueous solution of -15 to -20 mV, wherein the HGF is contained at a ratio of not less than 0.7 mol and not more than 7 mol per one mol of the gelatin.

4. An HGF gelatin complex comprising an HGF at a ratio of not less than 0.7 mol and not more than 7 mol per one mol of a gelatin having the following properties:
(1) an acidic gelatin obtained from collagen by an alkali hydrolysis treatment,
(2) a molecular weight of 100,000 - 200,000 daltons under non-reducing conditions of SDS-PAGE,
(3) an isoelectric point of about 5, and
(4) a zeta potential in an aqueous solution of -15 to -20 mV.

5. An aqueous HGF stabilized gelatin preparation comprising the HGF gelatin complex of claim 4.

6. An angiogenesis and vasculogenesis promoter comprising a preparation selected from the group consisting of the sustained release HGF gelatin hydrogel preparations of claims 1 to 2, the HGF stabilized gelatin preparation of claim 3, the HGF gelatin complex of claim 4 and the aqueous HGF stabilized gelatin preparation of claim 5.

7. A therapeutic agent for treating arterial diseases, which comprises a preparation selected from the group consisting of the sustained release HGF gelatin hydrogel preparations of claims 1 to 2, the HGF stabilized gelatin preparation of claim 3, the HGF gelatin complex of claim 4 and the aqueous HGF stabilized gelatin preparation of claim 5.

8. A therapeutic agent for treating organ disorders, which comprises a preparation selected from the group consisting of the sustained release HGF gelatin hydrogel preparations of claims 1 to 2, the HGF stabilized gelatin preparation of claim 3, the HGF gelatin complex of claim 4 and the aqueous HGF stabilized gelatin preparation of claim 5.

## Patentansprüche

1. HGF-Gelatine-Hydrogelpräparat mit nachhaltiger Wirkstofffreisetzung, das ein Gelatine-Hydrogel umfasst, welches durch Vernetzen einer Gelatine, die die folgenden Eigenschaften aufweist, und eines Hepatocyten-Wachstumsfaktors (HGF) erhalten wird:
(1) saure Gelatine, erhalten aus Collagen durch eine alkalische Hydrolysebehandlung;
(2) Molekulargewicht von 100 000 bis 200 000 Dalton unter nichtreduzierenden Bedingungen einer SDS-PAGE;
(3) isoelektrischer Punkt von etwa 5; und
(4) Zetapotential in einer wässrigen Lösung von -15 bis -20 mV;
wobei der HGF in einem Verhältnis von nicht weniger als 0,7 mol und nicht mehr als 7 mol pro Mol der Gelatine, die das Gelatine-Hydrogel bildet, enthalten ist.

2. HGF-Gelatine-Hydrogelpräparat mit nachhaltiger Wirkstofffreisetzung gemäß Anspruch 1, wobei das Gelatine-Hydrogel einen Wassergehalt von 80 bis 99 Gew.-% hat.

3. HGF-stabilisiertes Gelatinepräparat, das einen HGF und eine Gelatine mit den folgenden Eigenschaften umfasst:
(1) saure Gelatine, erhalten aus Collagen durch eine alkalische Hydrolysebehandlung;
(2) Molekulargewicht von 100 000 bis 200 000 Dalton unter nichtreduzierenden Bedingungen einer SDS-PAGE;
(3) isoelektrischer Punkt von etwa 5; und
(4) Zetapotential in einer wässrigen Lösung von -15 bis -20 mV;
wobei der HGF in einem Verhältnis von nicht weniger als 0,7 mol und nicht mehr als 7 mol pro Mol der Gelatine enthalten ist.

4. HGF-Gelatine-Komplex, der einen HGF in einem Verhältnis von nicht weniger als 0,7 mol und nicht mehr als 7 mol pro Mol einer Gelatine mit den folgenden Eigenschaften umfasst:
(1) saure Gelatine, erhalten aus Collagen durch eine alkalische Hydrolysebehandlung;
(2) Molekulargewicht von 100 000 bis 200 000 Dalton unter nichtreduzierenden Bedingungen einer SDS-PAGE;
(3) isoelektrischer Punkt von etwa 5; und
(4) Zetapotential in einer wässrigen Lösung von -15 bis -20 mV.

5. Wässriges HGF-stabilisiertes Gelatinepräparat, das den HGF-Gelatine-Komplex von Anspruch 4 umfasst.

6. Angiogenese- und Vasculogenese-Promotor, der ein Präparat umfasst, das aus der Gruppe ausgewählt ist, die aus den HGF-Gelatine-Hydrogelpräparaten mit nachhaltiger Wirkstofffreisetzung gemäß Anspruch 1 bis 2, dem HGF-stabilisierten Gelatinepräparat gemäß Anspruch 3, dem HGF-Gelatine-Komplex gemäß Anspruch 4 und dem wässrigen HGF-stabilisierten Gelatinepräparat gemäß Anspruch 5 besteht.

7. Therapeutisches Mittel zur Behandlung von Arterienkrankheiten, das ein Präparat umfasst, welches aus der Gruppe ausgewählt ist, die aus den HGF-Gelatine-Hydrogelpräparaten mit nachhaltiger Wirkstofffreisetzung gemäß Anspruch 1 bis 2, dem HGF-stabilisierten Gelatinepräparat gemäß Anspruch 3, dem HGF-Gelatine-Komplex gemäß Anspruch 4 und dem wässrigen HGF-stabilisierten Gelatinepräparat gemäß Anspruch 5 besteht.

8. Therapeutisches Mittel zur Behandlung von Organstörungen, das ein Präparat umfasst, welches aus der Gruppe ausgewählt ist, die aus den HGF-Gelatine-Hydrogelpräparaten mit nachhaltiger Wirkstofffreisetzung gemäß Anspruch 1 bis 2, dem HGF-stabilisierten Gelatinepräparat gemäß Anspruch 3, dem HGF-Gelatine-Komplex gemäß Anspruch 4 und dem wässrigen HGF-stabilisierten Gelatinepräparat gemäß Anspruch 5 besteht.

## Revendications

1. Préparation d'hydrogel de gélatine et d'HGF à libération prolongée comprenant un hydrogel de gélatine obtenu en réticulant une gélatine ayant les propriétés suivantes et un facteur de croissance des hépatocytes (HGF) :
(1) une gélatine acide obtenue à partir de collagène par un traitement d'hydrolyse alcaline,
(2) une masse moléculaire de 100 000 à 200 000 daltons dans des conditions non-réductrices de SDS-PAGE,
(3) un point isoélectrique d'environ 5, et
(4) une potentiel Zéta dans une solution aqueuse situé de -15 à -20 mV, dans laquelle le HGF est présent dans un rapport qui n'est pas inférieur à 0,7 mole et n'est pas supérieur à 7 moles par mole de la gélatine constituant l'hydrogel de gélatine.

2. Préparation d'hydrogel de gélatine et d'HGF à libération prolongée selon la revendication 1, dans laquelle l'hydrogel de gélatine présente une teneur en eau de 80 à 99 % p/p.

3. Préparation de gélatine stabilisée et d'HGF comprenant un HGF et une gélatine ayant les propriétés suivantes :
(1) une gélatine acide obtenue à partir de collagène par un traitement d'hydrolyse alcaline,
(2) une masse moléculaire de 100 000 à 200 000 daltons dans des conditions non-réductrices de SDS-PAGE,
(3) un point isoélectrique d'environ 5, et
(4) une potentiel Zéta dans une solution aqueuse de -15 à -20 mV, dans laquelle le HGF est présent dans un rapport qui n'est pas inférieur à 0,7 mole et n'est pas supérieur à 7 moles par mole de la gélatine.

4. Complexe gélatine-HGF comprenant un HGF à un rapport qui n'est pas inférieur à 0,7 mole et n'est pas supérieur à 7 moles par mole d'une gélatine ayant les propriétés suivantes :
(1) une gélatine acide obtenue à partir de collagène par un traitement d'hydrolyse alcaline,
(2) une masse moléculaire de 100 000 à 200 000 daltons dans des conditions non-réductrices de SDS-PAGE,
(3) un point isoélectrique d'environ 5, et
(4) une potentiel Zéta dans une solution aqueuse de -15 à -20 mV.

5. Préparation de gélatine stabilisée et d'HGF aqueux comprenant le complexe HGF-gélatine selon la revendication 4.

6. Promoteur de l'angiogenèse et de la vasculo-genèse comprenant une préparation choisie parmi le groupe constitué par les préparations d'hydrogel de gélatine et d'HGF à libération prolongée selon les revendications 1 à 2, la préparation de gélatine stabilisée et d'HGF selon la revendication 3, le complexe gélatine-HGF selon la revendication 4 et la préparation de gélatine stabilisée et d'HGF aqueux selon la revendication 5.

7. Agent thérapeutique pour le traitement des maladies artérielles, qui comprend une préparation choisie parmi le groupe constitué par les préparations d'hydrogel de gélatine et d'HGF à libération prolongée selon les revendications 1 et 2, la préparation de gélatine stabilisée et d'HGF selon la revendication 3, le complexe gélatine-HGF selon la revendication 4 et la préparation de gélatine stabilisée et d'HGF aqueux selon la revendication 5.

8. Agent thérapeutique pour le traitement des troubles des organes, qui comprend une préparation choisie parmi le groupe constitué par les préparations d'hydrogel de gélatine et d'HGF à libération prolongée selon les revendications 1 et 2, la préparation de gélatine stabilisée et d'HGF selon la revendication 3, le complexe gélatine-HGF selon la revendication 4 et la préparation de gélatine stabilisée et d'HGF aqueux selon la revendication 5.
